# EUROPÄISCHE PATENTSCHRIFT

(11) **EP 3 533 064 B1**
(45) Veröffentlichungstag und Bekanntmachung des Hinweises auf die Patenterteilung: **02.04.2025**
(21) Anmeldenummer: 17791674.9
(22) Anmeldetag: 27.10.2017
(51) Int. Cl.: G16H 20/40, G16H 10/40, G16H 10/65, G16H 10/60, G16H 40/40, G16H 40/63, A61M 1/14

(54) **MEDIZINISCHES BEHANDLUNGSSYSTEM**
MEDICAL TREATMENT SYSTEM
SYSTÈME DE TRAITEMENT MÉDICAL

(30) Priorität: 28.10.2016 DE 102016221337
(43) Veröffentlichungstag der Anmeldung: 04.09.2019
(73) Patentinhaber: Fresenius Medical Care Deutschland GmbH, 61352 Bad Homburg (DE)
(72) Erfinder: SCHERMEIER, Olaf, 60320 Frankfurt am Main (DE); KOULECHOV, Kirill, Shanghai 201204 (CN); EIFLER, Peter, 60594 Frankfurt am Main (DE)
(74) Vertreter: RCD-Patent Giesen, Schmelcher & Griebel Patentwanwälte PartG mbB
(86) Internationale Anmeldenummer: PCT/EP2017/077604
(87) Internationale Veröffentlichungsnummer: WO 2018/078099

(56) Entgegenhaltungen:
- WO-A2-2006/086735
- WO-A2-2007/051118
- WO-A2-2014/001970
- DE-A1- 102012 012 350
- US-A1- 2011 209 212
- US-A1- 2014 194 817
- US-A1- 2014 288 947
- US-A1- 2014 359 715

## Beschreibung

In vielen Bereichen der Medizintechnik ist die Sicherheit von Patienten ein großes Problem.

Dabei ist zudem festzustellen, dass wiederkehrende Behandlungen aus Kostengründen an Behandlungseinrichtungen verlagert werden, in denen häufig eine Vielzahl von Patienten mit ähnlichen Behandlungen versorgt werden können. Beispielhaft sei auf Dialysezentren verwiesen, in denen eine Vielzahl von Patienten mit unterschiedlichen Formen der Blutreinigung, wie z.B. Hämodialyse, Hämodiafiltration, Peritonealdialyse, Hämoultrafiltration, etc. behandelt werden können.

Bei solchen Behandlungen muss sichergestellt werden, dass ein Patient nur die passende Behandlung erhält.

Aus der US Patentanmeldung US 2014 / 194 817 A1 eine medizinische Pumpe für ein intravenöse Zuführung eines Medikamentes bekannt, bei der bestimmte Parameter nur nach Eingabe einer Autorisierung geändert werden können.

Aus der US Patentanmeldung US 2011 / 209 212 A1 ist ein Apheresesystem mit zwei Computern bekannt. Ein erster Computer agiert als Controller ein zweiter Computer agiert als Validierungseinrichtung.

Aus der US Patentanmeldung US 2014 / 288 947 A1 ist ein System und Verfahren für die Kommunikation mit einem Dialysegerät durch ein Netzwerk offenbart.

Aus der deutschen Patentanmeldung DE 10 2012 012350 A1 ist eine Vorrichtung und ein Verfahren zur Erzeugung und Anzeige von für medizinische Geräte und medizinische Behandlungen spezifischer Grafikkodierungen bekannt.

Bisher mussten die Behandlungsdaten eingegeben werden. Dies ist jedoch zeitaufwändig und lässt eine Fehleingabe von Parametern zu. D.h. eine fehlerhafte Eingabe konnte dazu führen, dass für patientenungeeignete Parameter eingestellt wurden. In der Folge können Schockzustände oder schwerwiegender Schädigungen auftreten.

### Aufgabe

Es ist daher eine Aufgabe der Erfindung ein System und Verfahren hierfür bereitzustellen, mit dem eine medizinische Behandlung mit erhöhter Sicherheit für den Patienten zur Verfügung gestellt werden kann.

### Kurzdarstellung der Erfindung

Die Aufgabe wird durch ein medizinisches Behandlungssystem gelöst. Das medizinische Behandlungssystem weist ein medizinisches Behandlungsgerät und ein Therapiefreigabe-Gerät auf.

Das medizinische Behandlungsgerät weist Eingabemittel zur Auswahl einer Behandlung auf, wobei das medizinische Behandlungsgerät weiterhin ein Gerät zur Erzeugung eines Patienten-und einer Behandlung-bezogenen Anforderungscodes aufweist, wobei der Anforderungscode Patientenidentitätsdaten aufweist, die eine eindeutige Identifikation des Patienten ermöglichen, wobei der Anforderungscode am medizinischen Behandlungsgerät auf einem Bildschirm zur Verfügung gestellt wird.

Das Therapiefreigabe-Gerät weist eine Freigabecodeerzeugungseinrichtung auf, wobei die Freigabecodeerzeugungseinrichtung ein Computer oder ein Microprozessor, ein Microcontroller, ein entsprechend eingerichteter ASIC (Application Specific Integrated Circuit) oder ein FPGA (Field Programmable Gate Array) ist, wobei das Therapiefreigabe-Gerät eine Einrichtung zum Empfang der Daten des Anforderungscodes aufweist, wobei der Anforderungscode durch einen Benutzer mittels eines Smartphones vom Bildschirm abgelesen oder eingelesen und mittels des Smartphones an das Therapiefreigabe-Gerät gesendet werden kann, wobei weiterhin die Freigabecodeerzeugungseinrichtung des Therapiefreigabe-Gerätes in Abhängigkeit von dem erhaltenen Anforderungscode und gespeicherten Daten entscheidet, ob ein Freigabecode erzeugt wird und den erzeugten Freigabecode sendet.

Das medizinische Behandlungsgerät weist weiterhin Mittel zum Einlesen eines Freigabecodes auf, wobei in Anhängigkeit vom Freigabecode und eventuell dem zuvor erzeugten Anforderungscode entschieden wird, ob die angeforderte Behandlung freigegeben wird , und wobei das medizinische Behandlungsgerät so eingerichtet ist, dass nach Freigabe das medizinische Behandlungsgerät entsperrt wird, so dass danach die ausgewählte Behandlung durchgeführt werden kann.

Die Aufgabe wird weiterhin durch entsprechende Verfahren auf dem medizinischen Behandlungsgerät bzw. dem Therapiefreigabe-Gerät gelöst.

Weitere vorteilhafte Ausgestaltungen sind Gegenstand der abhängigen Ansprüche und der ausführlichen Beschreibung.

### Kurzdarstellung der Figuren

Nachfolgend wird die Erfindung näher unter Bezugnahme auf die Figuren erläutert werden. In diesen zeigt:
- Fig. 1: eine schematische Übersicht auf Systeme gemäß Ausführungsformen der Erfindung, und
- Fig. 2: schematische Ablaufpläne für Verfahren gemäß Ausführungsformen der Erfindung.

### Detaillierte Beschreibung

Nachfolgend wird die Erfindung eingehender unter Bezugnahme auf die Figur dargestellt werden. Dabei ist anzumerken, dass unterschiedliche Aspekte beschreiben werden, die jeweils einzeln oder in Kombination zum Einsatz kommen können. D.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel immer nur auf eine Entität Bezug genommen werden. Soweit nicht explizit vermerkt, kann die Erfindung aber auch jeweils mehrere der betroffenen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird.

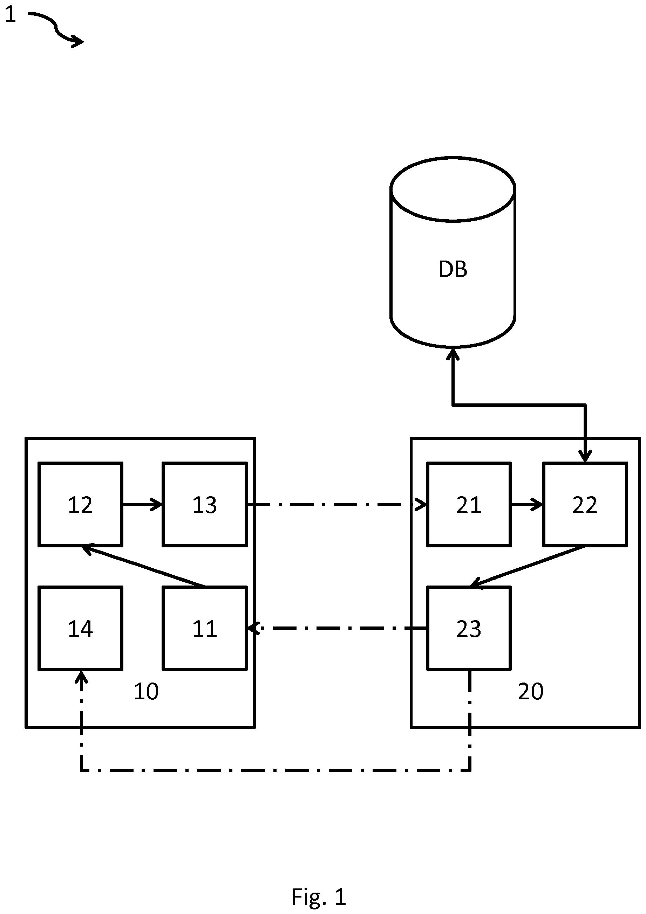

In Figur 1 ist ein medizinisches Behandlungssystem 1 schematisch dargestellt. Das medizinische Behandlungssystem 1 weist ein medizinisches Behandlungsgerät 10 und ein Therapiefreigabe-Gerät 20 auf. Obwohl nachfolgend sowohl das medizinische Behandlungsgerät 10 als auch das Therapiefreigabe-Gerät 20 als Einheit dargestellt werden, ist dies keine notwendige Eigenschaft, sondern jedes Gerät kann auch modular oder räumlich verteilt aufgebaut sein.

Das medizinische Behandlungsgerät weist Eingabemittel 11 zur Auswahl einer Behandlung auf. Eingabemittel können dabei z.B. typische Eingabemittel durch Schalter, Taster, Drehwähler, grafische Oberflächen eines Touchscreens, Tastaturen, Trackballs, Maussteuerung, etc., aber auch die Eingabe mittels Smartcard-Readers, etc. sein. D.h. es kommt lediglich darauf an, dass Daten in Bezug auf einen Patienten und eine für den Patienten angeforderte Behandlung dem medizinischen Behandlungsgerät 10 zur Verfügung gestellt werden können. Es sei dabei angemerkt, dass es auch möglich ist z.B. Patientendaten mit einem ersten Eingabemittel und angeforderte Behandlungsdaten mit einem zweiten Eingabemittel 11 einzugeben. Die Eingabe kann durch den Patienten und/oder durch medizinisch qualifiziertes Fach- oder Hilfspersonal erfolgen.

Das medizinische Behandlungsgerät 10 weist zudem ein Gerät 12 zur Erzeugung eines Patienten- und einer Behandlung-bezogenen Anforderungscodes auf. Beispielsweise kann das Gerät 12 zur Erzeugung eines Patienten- und Behandlung-bezogenen Anforderungscodes ein Computer oder ein Microprozessor, ein Microcontroller, ein entsprechend eingerichteter ASIC (Application Specific Integrated Circuit) oder ein FPGA (Field Programmable Gate Array) sein. Der Anforderungscode weist Patientenidentitätsdaten auf, die eine eindeutige Identifikation des Patienten ermöglichen.

Der so generierte Anforderungscode wird am medizinischen Behandlungsgerät 10 auf einem Bildschirm (13) zur Verfügung gestellt. Die Bereitstellung kann dabei auf verschiedenste Art und Weise zur Verfügung gestellt werden. Hierzu später.

Der generierte Anforderungscode wird sodann an das Therapiefreigabe-Gerät 20 übermittelt.

Der Patient oder das medizinisch qualifizierte Fach- oder Hilfspersonal kann den Code mittels eines Smartphones ablesen/einlesen und (z.B. mittels einer speziellen Applikation) an das Therapiefreigabe-Gerät 20 senden.

Das Therapiefreigabe-Gerät 20 weist wiederum eine Einrichtung zum Empfang 21 der Daten des Anforderungscodes auf. Die Einrichtung zum Empfang 21 kann z.B. mittels einer Datenschnittstelle, wie z.B. einer Netzwerk- und/oder Modemschnittstelle oder aber Eingabemittel - wie bereits zuvor in Zusammenhang mit dem medizinischen Behandlungsgerät 10 besprochen - aufweisen.

Das medizinische Behandlungsgerät 10 und das Therapiefreigabe-Gerät 20 sind dazu räumlich getrennt voneinander angeordnet. Beispielsweise kann ein Therapiefreigabe-Gerät 20 für eine Vielzahl von medizinischen Behandlungsgeräten 10 Dienste - wie nachfolgend beschrieben - zur Verfügung stellen.

Das Therapiefreigabe-Gerät 20 weist weiterhin eine Freigabecodeerzeugungseinrichtung 22 auf, welche in Abhängigkeit von einem erhaltenen Anforderungscode und gespeicherten Daten, die Patienten- und/oder Behandlungs-bezogen in einer lokalen oder entfernten Datenbank DB gespeichert sind und von dort abgerufen werden können, entscheidet, ob ein Freigabecode erzeugt wird, und den so erzeugten Anforderungscode mittels einer geeigneten Sendevorrichtung 23 sendet. Die Freigabecodeerzeugungseinrichtung 22 ist ein Computer oder ein Microprozessor, ein Microcontroller, ein entsprechend eingerichteter ASIC (Application Specific Integrated Circuit) oder ein FPGA (Field Programmable Gate Array).

Die Sendevorrichtung 23 kann z.B. mittels einer Datenschnittstelle, wie z.B. eine Netzwerk- und/oder Modem-Schnittstelle aufweisen. Beispielsweise können die Sendevorrichtung 23 und die Einrichtung zum Empfang 21 Teile einer gemeinsamen Datenschnittstelle sein.

Die Daten können dann direkt an das medizinische Behandlungsgerät 10 gesendet werden oder aber an ein Gerät, das in räumlicher Nähe hierzu befindlich ist.

Beispielsweise kann der Freigabecode an den Patienten oder das medizinisch qualifizierte Fach- oder Hilfspersonal, das den Anforderungscode gesendet hat oder aber in der Datenbank hinterlegt ist, gesendet werden.

Am medizinischen Behandlungsgerät 10 sind weiterhin Mittel zum Einlesen eines Freigabecodes 11 bzw. 14 vorgesehen, wobei in Anhängigkeit vom Freigabecode und eventuell dem zuvor erzeugten Anforderungscode durch das medizinische Behandlungsgerät 10 entschieden wird, ob die angeforderte Behandlung freigegeben wird.

D.h. mittels des Anforderungscodes, in dem der Patient und die angeforderte Behandlung codiert sind, wird das Therapiefreigabegerät in die Lage versetzt, die angeforderte Behandlung mit den Patientendaten und den hinterlegten Daten zu vergleichen. Dabei kann z.B. sichergestellt werden, dass die angeforderten Behandlungsdaten mit den für einen Patienten hinterlegten Daten übereinstimmen, z.B. dass ein bestimmter Behandlungsplan eingehalten wird, oder aber, dass die angeforderte Behandlung innerhalb einer bestimmten Variationsbreite der hinterlegten Daten befindlich ist.

D.h. es kann sichergestellt werden, dass ein Patient nur die passende Behandlung erhält.

Dabei können Fehleingaben vermieden werden, wodurch die Patientensicherheit erhöht wird.

Zudem erlaubt das System auch die Protokollierung der angeforderten Behandlungen, sodass z.B. ein Therapieverlauf ausgewertet werden kann. Somit wird die Möglichkeit einer Qualitätssicherung geschaffen.

Andere Parameter, die Patienten- und/oder Behandlungs-bezogen sind, können zudem in die Freigabeentscheidung mit einbezogen werden.

In einer Ausgestaltung der Erfindung weist der Anforderungscode Daten ausgewählt aus einer Gruppe aufweisend: Patientenzustandsdaten, Behandlungsparameter, Geräteparameter, Parameter von Verbrauchsmaterialien auf.

Patientenidentitätsdaten können z.B. Identifikationsnummern, wie z.B. Sozial- / Krankenversicherungsnummer, Name, Vorname, Geburtsdaten etc. sein, die eine eindeutige Identifikation des Patienten ermöglichen. Entsprechende Daten werden auch in der lokalen oder entfernten Datenbank DB gespeichert, um eine Zuordnung von Datensätzen zu ermöglichen.

Patientenzustandsdaten können z.B. Gewicht, Größe, Herzfrequenz, Blutdruck, Blutzucker, Hämoglobin, Hämatokrit, relatives Blutvolumen, Lungenvolumen, etc. umfassen. D.h. alle Patientenzustandsdaten, die für eine Behandlung von Relevanz sind, können ebenfalls übermittelt werden.

Behandlungsparameter sind z.B. die angeforderte Art einer Behandlung, wie z.B. Hämodiafiltration, Hämodialyse, Ultrafiltration etc. Beispielsweise lassen einige medizinische Behandlungsgeräte 10 eine Vielzahl von Behandlungen zu, sodass es wichtig ist, die angeforderte Behandlung (z.B. Hämodialyse) zu übermitteln, sowie gegebenenfalls die jeweiligen Parameter der ausgewählten Behandlung, wie z.B. Dauer der Behandlung, eingestellter Blutpumpenrate eingestellte Dialysatpumpenrate, Substitutionsvolumen und - rate, Ultrafiltrationsvolumen und -rate, etc..

Geräteparameter sind z.B. Zustands- und/oder Geräteeigenschaftsdaten (Art der Maschine, Identifikationsnummer, Ausstattung), die es dem Therapiefreigabe-Gerät 20 erlauben zu entscheiden, ob eine angeforderte Behandlung auch mit dem Gerät sinnvoll (noch) ausgeführt werden kann.

Parameter von Verbrauchsmaterialien können z.B. Daten in Bezug auf Dialysierflüssigkeiten (Art und Zusammensetzung der Dialysierflüssigkeit und/oder Substitutionsflüssigkeit), Dialysatoren, verwendete Einmalartikel wie Schlauchsets, Dialysefilter, etc. sein.

Im Allgemeinen umfassen die Daten zumindest so viele charakteristische Merkmale, dass eine Behandlung eindeutig einem Patienten zuzuordnen ist.

In einer Ausgestaltung der Erfindung wird der Anforderungscode graphisch codiert am medizinischen Behandlungsgerät 10 bereitgestellt, z.B. auf einem Bildschirm.

Dabei kann der Anforderungscode beispielsweise aus einer Gruppe aufweisend QR-Code, Barcode, Flickercode ausgewählt sein. Dabei können ein- oder mehrdimensionale Codes Verwendung finden.

D.h. der Anforderungscode kann z.B. auf einem Bildschirm 13 am medizinischen Behandlungsgerät 10 zur Verfügung gestellt werden. Beispielsweise kann der Patient oder das medizinisch qualifizierte Fach- oder Hilfspersonal den Anforderungscode mittels eines Smartphones (z.B. mittels einer speziellen Applikation) ablesen/einlesen und an das Therapiefreigabe-Gerät 20 senden. Dabei kann die Mobilfunknummer oder MAC-Adresse des Smartphone als (zusätzliche) Identifikation des Patienten oder des medizinisch qualifizierten Fach- oder Hilfspersonals dienen. D.h. die Handhabung wird stark vereinfacht.

In einer Ausgestaltung der Erfindung weist der Anforderungscode eine Verschlüsselung z.B. als Secure QR-Code auf. Hierdurch wird zum einen die Datensicherheit in Bezug auf personenbezogene Daten des Patienten als auch die Sicherheit der Datenkommunikation insgesamt verbessert.

In einer weiteren Ausgestaltung ist das Eingabemittel 11 bzw. 14 ausgewählt aus einer Gruppe aufweisend Kartenleser, Graphische Oberfläche und Nahfeldkommunikationsleser. D.h. es können mit einer Vielzahl von Technologien Daten eingelesen werden, sodass z.B. eine Interaktion mit der in Deutschland eingeführten Gesundheitskarte (Kartenleser) über einen Kartenleser 14 als auch mit einem elektronischen Ausweisdokument (NFC, RFID) oder einem Smartphone (NFC, Bluetooth, ZigBee, WLAN) ebenso möglich ist, wie die Eingabe auf einem Touchscreen 11. D.h. es können alternativ oder zusätzlich zu einer graphischen Kodierung auch über Nahfeldkommunikation (NFC, RFID) von dem medizinischen Behandlungsgerät 10 z.B. zu einem mobilen Gerät und umgekehrt Daten ausgetauscht werden. Dies macht das Scannen und die manuelle Eingabe des Freischaltcodes überflüssig.

In einer weiteren Ausgestaltung wird im Falle, dass eine angeforderte Behandlung abgelehnt wird als Freigabecode eine Aufforderung zur Abgabe eines qualifizierten Anforderungscodes durch medizinisch geschultes Personal versandt.

D.h. wenn z.B. eine Behandlung außerhalb der vorgesehen Parameter ist, dies aber durch eine lokale Prüfung sinnvoll erscheint, soll die Freigabe ermöglicht werden. Andererseits wird durch die Rückmeldung ein möglicher Eingabefehler für den Patienten und/oder das medizinische Fach- oder Hilfspersonal vor Ort sichtbar oder aber eine Überprüfung des Gesundheitszustandes veranlasst, sollte z.B. Patientenzustandsdaten eine Behandlung mittels der angeforderten Behandlung kontraindizieren. Erst durch Eingabe eines qualifizierten Anforderungscodes kann dann die Behandlung freigegeben werden.

In einer vorteilhaften Ausgestaltung kann z.B. vorgesehen sein, dass der qualifizierte Anforderungscode weiterhin die Identifikation als medizinisch geschultes Personal aufweist.

Insbesondere kann der qualifizierte Anforderungscode weiterhin die Identifikation des medizinisch geschulten Personals ausgewählt aus einer Gruppe Fingerscan, Irisscan, PIN aufweisen. Hierzu kann z.B. auch eine optische Einleseeinrichtung als Eingabemittel 11 vorgesehen sein. Natürlich kann in gleicher Weise auch ein Patient identifiziert werden. Es sei dabei angemerkt, dass z.B. vorgesehen sein kann, das mit dem Grad einer Abweichung von vorbestimmten Werten/Wertebereichen der Grad der Qualifizierung steigt, sodass z.B. bei geringen Abweichungen das lokale medizinische Hilfspersonal eine Freigabe durch einen qualifizierten Anforderungscode (lokal oder mit Hilfe des Therapiefreigabe-Geräts 20) ermöglichen kann, während bei größeren Abweichungen nur das lokale medizinische Fachpersonal eine Freigabe durch einen qualifizierten Anforderungscode (lokal oder mit Hilfe des Therapiefreigabe-Geräts 20) ermöglichen kann.

In einer Ausgestaltung der Erfindung werden (qualifizierte) Anforderungscodes und / oder Freigabecode mittels eines drahtlosen oder drahtgebundenen Kommunikationsnetzes ausgetauscht. Beispielhafte Kommunikationsnetze sind LANs (Local Area Networks) WANs (Wide Area Networks) als auch WLANs (Wireless LANs) als auch Mobilfunknetze wie z.B. LTE,UMTS/GSM-Netze.

In einer weiteren Ausgestaltung der Erfindung kann z.B. für Notfälle oder bei Fehlen einer Zugriffsmöglichkeit auf das Therapiefreigabe-Gerät 20 es vorteilhaft sein, das eine angeforderte Behandlung lokal freigegeben werden kann, wobei zur lokalen Freigabe z.B. die Eingabe einer Emergency-TAN erforderlich ist. D.h. ist beispielsweise ein Notfall eingetreten und/oder das Therapiefreigabe-Gerät 20 nicht erreichbar, so kann durch das lokale medizinisch qualifizierte Personal dennoch eine (dringend) benötigte Behandlung als Ausnahmefall freigegeben werden. Dabei können vorgesehen sein, dass z.B. nur ein eingeschränkter Notfallbetrieb möglich ist, und nicht die volle Bandbreite an Behandlungen zur Verfügung steht. Außerdem kann vorgesehen sein, dass bestimmte Behandlungen nur durch medizinisches Fachpersonal freigegeben werden können.

In einer weiteren Ausgestaltung der Erfindung kann zudem vorgesehen sein, dass die Freigabecodeerzeugungseinrichtung 22 in Abhängigkeit von einem erhaltenen Anforderungscode und gespeicherten Daten entscheidet, ob eine angeforderte Behandlung für den Patienten plausibel ist und ein Freigabecode nur dann erzeugt wird, wenn die angeforderte Behandlung für den Patienten plausibel ist. Die Prüfung der Plausibilität kann den Vergleich der angeforderten Behandlung mit vorangegangenen Behandlungen eines konkreten Patienten umfassen. Weichen die Behandlungen bzw. deren Parameter stark voneinander ab und/oder bzw. wenn die Behandlung medizinischen Leitlinien widerspricht die angeforderte Behandlung nicht plausibel ist, kann gefolgert werden, dass die vorliegende Behandlungsanforderung nicht plausibel ist, und ein Freigabecode wird zunächst nicht erzeugt. Die Prüfung der (medizinischen) Plausibilität kann von einem Expertensystem vorgenommen werden, dass im Therapiefreigabe-Gerät 20 oder einem weiteren Gerät, welches mit dem Therapiefreigabe-Gerät 20 interagieren kann, vorgehalten ist.

Ohne Beschränkung der Allgemeinheit ist die Erfindung auf eine Vielzahl von unterschiedlichen Behandlungssystemen anwendbar. Insbesondere ist jedoch das medizinische Behandlungsgerät 10 ein Blutbehandlungsgerät ausgewählt aus der Gruppe aufweisend insbesondere Dialysegerät, Plasmapheresegerät, Apheresegerät, Hämodiafiltration, Hämoultrafiltration, Hämoperfusion, Peritonealdialyse, Albumin Dialyse.

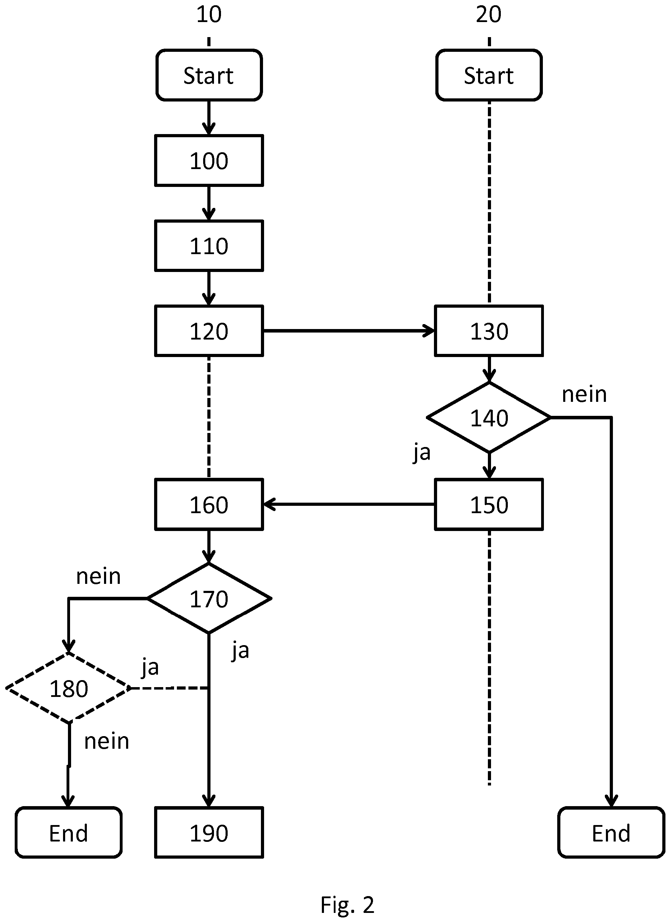

Ohne Weiteres ergibt sich aus der vorherigen Beschreibung auch im Hinblick auf die Figur 2 eine mögliche Ausgestaltung der Verfahren.

In einem ersten Schritt 100 wird am medizinischen Behandlungsgerät eine Behandlung und gegebenenfalls Behandlungsparameter ausgewählt. Zusammen mit Patientendaten, die ebenfalls eingegeben oder eingelesen werden, wird in Schritt ein Patienten- und Behandlungsbezogenen Anforderungscode erzeugt und Bereitstellen des Anforderungscodes in Schritt 120 durch das medizinische Behandlungsgerät 10 bereitgestellt.

Der bereitgestellte Anforderungscode wird an das Therapiefreigabe-Gerät 20 übermittelt und von diesem in Schritt 130 empfangen. Anschließend wird in Schritt 140 in Abhängigkeit von dem erhalten Anforderungscode und gespeicherten Daten - abgerufen aus der Datenbank DB - entschieden, ob ein Freigabecode erzeugt wird. Im Falle, dass ein Freigabecode erzeugt wird, wird der erzeugte Freigabecode in Schritt 150 durch das Therapiefreigabe-Gerät 20 in Richtung des medizinischen Behandlungsgeräts 10 gesandt. Optional kann der Freigabecode auch die Aufforderung zur qualifizieren Freigabe enthalten. Wird die Freigabe nicht erteilt, kann das Programm terminieren. Alternativ oder zusätzlich kann auch ein "Freigabecode" erzeugt werden, der auf die Verweigerung und den Grund der Verweigerung der Freigabe hinweist. D.h. am medizinischen Behandlungsgerät 10 kann die entsprechende Fehlermeldung angezeigt werden.

Der versandte Freigabecode kann dann am medizinischen Behandlungsgerät 10 in Schritt 160 eingelesen/eingegeben oder ganz allgemein empfangen werden. Nunmehr kann durch das medizinische Behandlungsgerät 10 in Schritt 170 in Anhängigkeit vom Freigabecode und dem zuvor erzeugten Anforderungscode entschieden werden, ob die angeforderte Behandlung freigegeben wird.

Wird die angeforderte Behandlung freigegeben, so kann in Schritt 190 die Behandlung gestartet werden. Nachfolgend zu einer erfolgten Behandlung kann vorgesehen sein, dass dies dem Therapiefreigabe-Gerät 20 zur Protokollierung optional zusammen mit weiteren Verlaufsdaten der erfolgten Behandlung übermittelt wird.

Wird die angeforderte Behandlung nicht freigegeben, so können z.B. optional eine Notfall-Freigabe und/oder einer qualifizierte Freigabe in einem optionalen Schritt 180 vorgesehen sein. Hierzu kann z.B. vorgesehen sein, dass eine gewisse Anzahl von Behandlungen durch Eingabe einer TAN, z.B. aus einer Liste, ermöglicht wird und/oder eine TAN von einer Notfall-Hotline erhalten wird.

Wird die (qualifizierte) Freigabe nicht erteilt, kann das Programm terminieren.

Es kann vorgesehen sein, dass zwischen der Bereitstellung eines Anforderungscodes in Schritt 120 und dem Empfang eines Freigabecodes in Schritt 160 eine maximale Zeit nicht überschritten werden darf, um z.B. ein medizinisches Behandlungsgerät 10 bei Ausbleiben einer Antwort, z.B. in Folge von Kommunikationsproblemen oder Ausfall des Therapiefreigabe-Gerätes 20, nicht zu blockieren. D.h. nach Ablauf einer vorbestimmten Zeitspanne kann das medizinische Behandlungsgerät 10 automatisch einen fehlerhaften Freigabecode annehmen und in Schritt 180 die Eingabe erwarten.

Auch hier kann wiederum eine zeitliche Befristung vorgesehen sein, sodass das Verfahren sicher endet.

Die Erfindung kann auch in einem oder mehreren Computerprogrammprodukten zur Programmierung von Einrichtungen eines medizinischen Behandlungssystems zur Ausführung von Schritten eines Verfahrens verkörpert sein. Computerprogrammprodukte können dabei als herunterladbare Datenfolge oder aber als Datenträger, z.B. DVD, CD, Speicherkarte, Speicherchip zur Verfügung gestellt werden.

Nachfolgend werden wir die Erfindung für einen Anwendungsfall nochmals beschreiben.

Steht eine Behandlung eines Patienten an, werden die Behandlungsdaten in das medizinische Behandlungsgerät 10, beispielsweise das Dialysegerät, eingegeben. Zur Eingabe kann ein GUI (Graphical User Interface) verwendet werden. Patientendaten und/oder auch Behandlungsdaten können aber auch durch mobile Datenspeicher, wie Patientenkarten (Smart Cards) und geeignete Lesegeräte dem medizinischen Behandlungsgerät 10 mitgeteilt werden.

Eine im medizinischen Behandlungsgerät 10 vorgehaltene Software wandelt diese Daten in eine (verschlüsselte) graphische Kodierung. Diese Kodierung kann beispielsweise durch ein Smartphone eingelesen werden und über eine Software (App) entschlüsselt und dekodiert werden Die Daten werden hiernach an das Therapiefreigabe-Gerät 20 übermittelt (per Mobilfunknetz, Internet etc.), wo sie als Behandlungsanforderung geprüft werden.

Ergibt die Überprüfung der Daten, dass die angeforderte Behandlung bestimmten Kriterien entspricht, wird dem mobilen Gerät ein Freigabecode zurückgemeldet (TAN beispielsweise). Dieser Freigabecode erlaubt es, das medizinische Behandlungsgerät 10 zu entsperren, sodass danach die Behandlung durchgeführt werden kann.

Die Kriterien, die im Therapiefreigabe-Gerät 20 geprüft werden, können mannigfaltig sein.

Weiterhin kann aber auch dem Therapiefreigabe-Gerät 20 eine Expertensystem unterlagert sein, das weitere Kriterien überprüft, beispielsweise, ob die angeforderte Behandlung medizinisch schlüssig ist oder aber potentiell gefährliche Einstellungen für die Behandlung vorliegen. Medizinisch unschlüssig kann z.B. eine Behandlung werden, wenn Patientenzustandsdaten einer Behandlung aufgrund medizinischer Erwägungen widersprechen.

In einer Ausführungsform kann bei entsprechendem Ausgang der Kriterienprüfung statt eines Freigabecodes eine Fehler- oder Alarmmeldung in Schritt 150 zurückgesendet werden.

In einer Ausführungsform kann die Kriterienprüfung jedoch auch dazu führen, dass die Freigabecodeerzeugungseinrichtung in Abhängigkeit von einem erhalten Anforderungscode und gespeicherten Daten entscheidet, ob eine angeforderte Behandlung für den Patienten plausibel ist und, wenn die angeforderte Behandlung für den Patienten nicht plausibel ist, den Freigabecode um Daten ergänzt, sodass eine plausible Behandlung freigegeben werden kann. Diese Daten können absolute Daten als auch relative Änderungsdaten umfassen, sodass z.B. nur die Abweichung von Daten in Bezug auf die angeforderte Behandlung übermittelt werden. Hierdurch wird z.B. eine Fehleingabe erkannt (und möglicherweise auch durch eine Fehler- oder Alarmmeldung signalisiert) und sodann auch eine Behandlung, die medizinisch sinnvoll erscheint, vorschlägt. Die vorgeschlagene Behandlung kann sodann am medizinischen Behandlungsgerät 10 angezeigt (z.B. zusammen mit der Fehler- oder Alarmmeldung) werden. Anschließend kann dann durch das entsprechende Personal die geänderte Behandlung freigegeben werden. D.h das medizinische Behandlungsgerät 10 entscheidet zumindest in Abhängigkeit vom Freigabecode ob und wie die angeforderte Behandlung freigegeben wird.

In einer weiteren Ausführungsform kann in einem solchen Fall von qualifiziertem medizinischem Personal dennoch ein gültiger Freischaltcode beantragt werden, in dem das qualifizierte medizinische Personal sich beispielsweise durch die Eingabe einer Geheimnummer am mobilen Gerät identifiziert (z.B. mittels Fingerabdruckscanner, Irisscan oder sonstige biometrische Daten, die eine Person eindeutig identifizieren) und so nach erfolgter Prüfung der Qualifikation des Beantragenden trotz der vom Expertensystem angemeldeten Bedenken einen gültigen Freischaltcode erhält.

Hierbei hängt das "Überstimmen" der Bedenken des Expertensystems von der Qualifikation des Bedieners ab. Ärzte haben bspw. ein höheres Überstimmrecht als Krankenschwestern.

Vorteilhafterweise wird ein solcher Vorgang protokolliert, d.h. es ist jederzeit nachvollziehbar, von wem das Expertensystem überstimmt wurde, oder versucht wurde, das Expertensystem zu überstimmen.

In vorteilhafter Weise können alle Vorgänge im Zusammenhang mit der Freigabe der Behandlung protokolliert werden.

Der vom medizinischen Behandlungsgerät 10 zur Freigabe der Behandlung erwartete Freischaltcode kann in einer Ausführungsform durch eine Software aus den eingegebenen Daten berechnet werden. Ein Vorteil dieser Ausführungsform ist, dass das medizinische Behandlungsgerät 10 selbst keine datentechnische Verbindung nach außen benötigt, was die Datensicherheit erhöht. D.h. das Fehlen einer Schnittstelle zur direkten Kommunikation erhöht die Sicherheit des medizinischen Behandlungsgerätes 10.

Die Berechnung des Freischaltcodes kann in diesem Fall z.B. durch bestimmte Algorithmen (beispielsweise ein Key Generator) vorgenommen werden, die eindeutige Freischaltcodes (z.B. optisch codierte Codes wie QR-Code, Flicker-Code) aus den eingegebenen Daten erstellen.

In einer weiteren Ausführungsform, bei der das medizinische Behandlungsgerät 10 eine datentechnische Verbindungsmöglichkeit (Internet, Mobilfunk etc.) aufweist, kann der Freischaltcode zufällig vom Therapiefreigabe-Gerät 20 erzeugt werden und dem medizinischen Behandlungsgerät 10 über eine Datenübermittlung mitgeteilt werden.

Auch bei einem solchermaßen ausgestatteten medizinischen Behandlungsgerät 10 kann die beschriebene Anforderung eines Freischaltcodes über ein mobiles Gerät sinnvoll sein.

Hierbei fungiert das mobile Gerät quasi als Schlüssel für das medizintechnische Gerät, wobei der Schlüssel eindeutig zugeordnet werden kann (über beispielsweise die Übersendung von Geräteiidentifikationsnummern und die Eingabe von personenindividuellen PINs bzw. Eingabe biometrischer Daten). Nur qualifizierte Personen erhalten den Schlüssel, in dem die Software, die die oben beschriebene Funktionalität aufweist, personengebunden registriert werden muss.

## Patentansprüche

1. Medizinisches Behandlungssystem (1) aufweisend
ein medizinisches Behandlungsgerät (10), wobei das medizinische Behandlungsgerät Eingabemittel (11) zur Auswahl einer Behandlung aufweist, wobei das medizinische Behandlungsgerät ein Gerät (12) zur Erzeugung eines Patienten- und einer Behandlung-bezogenen Anforderungscodes aufweist, wobei der Anforderungscode Patientenidentitätsdaten aufweist, die eine eindeutige Identifikation des Patienten ermöglichen, wobei der Anforderungscode am medizinischen Behandlungsgerät (10) auf einem Bildschirm (13) zur Verfügung gestellt wird,
ein Therapiefreigabe-Gerät (20), wobei das Therapiefreigabe-Gerät (20) eine Freigabecodeerzeugungseinrichtung (22) aufweist, wobei die Freigabecodeerzeugungseinrichtung (22) ein Computer oder ein Microprozessor, ein Microcontroller, ein entsprechend eingerichteter ASIC (Application Specific Integrated Circuit) oder ein FPGA (Field Programmable Gate Array) ist, wobei das Therapiefreigabe-Gerät (20) eine Einrichtung zum Empfang (21) der Daten des Anforderungscodes aufweist, wobei der Anforderungscode durch einen Benutzer mittels eines Smartphones vom Bildschirm abgelesen oder eingelesen und mittels des Smartphones an das Therapiefreigabe-Gerät (20) gesendet werden kann, wobei weiterhin die Freigabecodeerzeugungseinrichtung (22) des Therapiefreigabe-Geräts (20) in Abhängigkeit von dem erhalten Anforderungscode und gespeicherten Daten (DB) entscheidet, ob ein Freigabecode erzeugt wird, und den erzeugten Freigabecode sendet (23),
• wobei das medizinische Behandlungsgerät (10) weiterhin Mittel zum Einlesen eines Freigabecodes (11, 14) aufweist,
• wobei das medizinische Behandlungsgerät (10) zumindest in Abhängigkeit vom Freigabecode entscheidet, ob und gegebenenfalls wie die angeforderte Behandlung freigegeben wird, und
• wobei das medizinische Behandlungsgerät (10) so eingerichtet ist, dass nach Freigabe das medizinische Behandlungsgerät (10) entsperrt wird, sodass danach die ausgewählte Behandlung durchgeführt werden kann.

2. Medizinisches Behandlungssystem nach Anspruch 1, wobei der Anforderungscode Daten aufweist ausgewählt aus einer Gruppe aufweisend: Patientenzustandsdaten, Behandlungsparameter, Geräteparameter, Parameter von Verbrauchsmaterialien.

3. Medizinisches Behandlungssystem nach einem der vorhergehenden Ansprüche, wobei der Anforderungscode ausgewählt ist aus einer Gruppe aufweisend QR-Code, Barcode, Flickrcode.

4. Medizinisches Behandlungssystem nach einem der vorhergehenden Ansprüche, wobei der Anforderungscode eine Verschlüsselung aufweist.

5. Medizinisches Behandlungssystem nach einem der vorhergehenden Ansprüche, wobei das Eingabemittel ausgewählt ist aus einer Gruppe aufweisend Kartenleser, Graphische Oberfläche, Nahfeldkommunikationsleser.

6. Medizinisches Behandlungssystem nach einem der vorhergehenden Ansprüche, wobei im Falle, dass eine angeforderte Behandlung abgelehnt wird, der Freigabecode eine Aufforderung zur Abgabe eines qualifizierten Anforderungscodes durch medizinisch geschultes Personal aufweist.

7. Medizinisches Behandlungssystem nach einem der vorhergehenden Ansprüche, wobei Anforderungscode und / oder Freigabecode mittels eines drahtlosen oder drahtgebundenen Kommunikationsnetzes ausgetauscht werden.

8. Medizinisches Behandlungssystem nach einem der vorhergehenden Ansprüche, wobei weiterhin am medizinischen Behandlungsgerät eine angeforderte Behandlung lokal freigegeben werden kann.

9. Medizinisches Behandlungssystem nach einem der vorhergehenden Ansprüche, wobei die Freigabecodeerzeugungseinrichtung in Abhängigkeit von einem erhalten Anforderungscode und gespeicherten Daten entscheidet, ob eine angeforderte Behandlung für den Patienten plausibel ist und ein Freigabecode nur dann erzeugt wird, wenn die angeforderte Behandlung für den Patienten plausibel ist.

10. Medizinisches Behandlungssystem nach Anspruch 9, wobei die Entscheidung, ob eine angeforderte Behandlung plausibel ist
• den Vergleich der angeforderten Behandlung mit vorangegangenen Behandlungen und/oder deren Parametern, wobei wenn die Behandlungen bzw. deren Parameter stark voneinander abweichen die angeforderte Behandlung nicht plausibel ist, und/oder
• die Prüfung der medizinischen Plausibilität durch ein Expertensystem, wobei wenn die Behandlung medizinischen Leitlinien widerspricht die angeforderte Behandlung nicht plausibel ist,
mit umfasst.

11. Medizinisches Behandlungssystem nach einem der vorhergehenden Ansprüche, wobei das medizinisches Behandlungsgerät (10) , ausgewählt aus der Gruppe aufweisend Dialysegerät, Plasmapheresegerät, Apheresegerät, Hämodiafiltration, Hämoultrafiltration, Hämoperfusion, Peritnoealdialyse, Albumin Dialyse ist.

12. Verfahren für ein Medizinisches Behandlungssystem (1) aufweisend ein medizinisches Behandlungsgerät (10) und ein Therapiefreigabe-Gerät (20), aufweisend die Schritte:
• Auswahl einer Behandlung (100) am Behandlungsgerät (10),
• Erzeugung eines Patienten- und einer Behandlung-bezogenen Anforderungscodes (110), wobei der Anforderungscode Patientenidentitätsdaten aufweist, die eine eindeutige Identifikation des Patienten ermöglichen, und Bereitstellen des Anforderungscodes (120) durch das Behandlungsgerät (10) auf einem Bildschirm (13),
• Einlesen des Anforderungscodes vom Bildschirm bzw. Eingeben des Anforderungscodes mittels eines Smartphones durch einen Benutzer und Senden des vom Bildschirm abgelesen oder eingelesen Anforderungscodes mittels des Smartphones an das Therapiefreigabe-Gerät (20),
• Empfangen des Anforderungscodes (130) am Therapiefreigabe-Gerät (20),
• Entscheiden (140) durch das Therapiefreigabe-Gerät (20) in Abhängigkeit von einem erhaltenen Anforderungscode und gespeicherten Daten, ob ein Freigabecode erzeugt wird, und
• Senden des erzeugten Freigabecodes (150) durch das Therapiefreigabe-Gerät (20),
• Empfangen des erzeugten Freigabecodes (160) am medizinischen Behandlungsgerät (10) über Mittel zum Einlesen eines Freigabecodes (11, 14),
• Entscheiden (170) durch das medizinische Behandlungsgerät (10) in Anhängigkeit vom zumindest dem Freigabecode und dem zuvor erzeugten Anforderungscode, ob und gegebenenfalls wie die angeforderte Behandlung freigegeben wird, wobei nach Freigabe das medizinische Behandlungsgerät (10) entsperrt wird, so dass danach die ausgewählte Behandlung durchgeführt werden.

13. Verfahren für ein medizinisches Behandlungsgerät (10) in einem medizinischen Behandlungssystem (1) aufweisend das medizinische Behandlungsgerät (10) und ein Therapiefreigabe-Gerät (20), aufweisend die Schritte:
• Erhalten einer Auswahl (100) einer Behandlung am medizinischen Behandlungsgerät (10),
• Erzeugung eines Patienten- und Behandlung-bezogenen Anforderungscodes (110), wobei der Anforderungscode Patientenidentitätsdaten aufweist, die eine eindeutige Identifikation des Patienten ermöglichen, und Bereitstellen des Anforderungscodes (120) durch das medizinische Behandlungsgerät (10) auf einem Bildschirm (13),
• Empfangen (160) eines vom Therapiefreigabe-Gerät (20) erzeugten Freigabecodes am medizinischen Behandlungsgerät (10) über Mittel zum Einlesen eines Freigabecodes (11, 14),
• Entscheiden (170) durch das medizinische Behandlungsgerät (10) in Anhängigkeit vom Freigabecode und dem zuvor erzeugten Anforderungscode, ob die angeforderte Behandlung freigegeben wird, wobei nach Freigabe das medizinische Behandlungsgerät (10) entsperrt wird, so dass danach die ausgewählte Behandlung durchgeführt werden kann.

14. Verfahren für ein Therapiefreigabe-Gerät (20) in einem medizinischen Behandlungssystem (1) aufweisend ein medizinisches Behandlungsgerät (10) mit Bildschirm (13) und das Therapiefreigabe-Gerät (20), aufweisend die Schritte:
• Empfangen (130) eines Anforderungscodes am Therapiefreigabe-Gerät (20), wobei der Anforderungscode Patientenidentitätsdaten aufweist, die eine eindeutige Identifikation des Patienten ermöglichen, wobei der Anforderungscode zuvor mittels eines Smartphones durch einen Benutzer vom Bildschirm (13) abgelesen oder eingelesen wurde, und der vom Bildschirm (13) abgelesene oder eingelesene Anforderungscode mittels des Smartphones an das Therapiefreigabe-Gerät (20) gesendet wurde,
• Entscheiden (140) in Abhängigkeit von einem erhaltenen Anforderungscode und gespeicherten Daten, ob ein Freigabecode erzeugt wird, und
• Senden (150) des erzeugten Freigabecode durch das Therapiefreigabe-Gerät (20).

15. Computerprogrammprodukt zur Programmierung von Einrichtungen eines medizinischen Behandlungssystems zur Ausführung von Schritten eines Verfahrens gemäß Anspruch 13.

16. Computerprogrammprodukt zur Programmierung von Einrichtungen eines medizinischen Behandlungssystems zur Ausführung von Schritten eines Verfahrens gemäß Anspruch 14.

## Claims

1. A medical treatment system (1), comprising
- a medical treatment device (10), wherein the medical treatment device comprises input means (11) for selecting a treatment, wherein the medical treatment device comprises a device (12) for generating a patient-related and a treatment-related request code, whereby the request code comprises patient identity data, which allows for a unique identification of the patient, wherein the request code is presented on a display (13) on the medical treatment device (10),
- a therapy-enabling device (20), wherein the therapy-enabling device (20) comprises an enabling code generating device (22) , whereby the enabling code generating device (22) is a computer or a microprocessor, a microcontroller, an appropriately configured ASIC (application-specific integrated circuit) or a FPGA (field programmable gate array), whereby the therapy-enabling device (20) comprises a device (21) for receiving the data of the request code, wherein the request code may be read or inputted by a user by means of a smartphone from the display and send to the therapy-enabling device (20) via the smartphone, whereby the enabling code generating device (22) of the therapy-enabling device (20) depending on the received request code and stored data (DB), decides whether an enabling code will be generated and sends the generated request code (23),
- wherein the medical treatment device (10) furthermore comprises means for entering an enabling code (11, 14),
- wherein the medical treatment device (10) decides, at least as a function of the enabling code, whether and if necessary how the requested treatment will be enabled, and
- whereby the medical treatment device (10) is arranged such that after enablement the medical treatment device (10) is unlocked, for that the selected treatment may be executed.

2. The medical treatment system according to claim 1, wherein the request code comprises data selected from a group comprising: patient condition data, treatment parameters, device parameters, parameters concerning consumables.

3. The medical treatment system according to one of the preceding claims, wherein the request code is selected from a group comprising QR codes, bar codes and flickr codes.

4. The medical treatment system according to one of the preceding claims, wherein the request code comprises an encryption.

5. The medical treatment system according to one of the preceding claims, wherein the input means is selected from a group comprising a card reader, graphical user interface, near field communication reader.

6. The medical treatment system according to one of the preceding claims, wherein in the case in which a requested treatment is declined, the enabling code comprises a request for issuing a qualified request code by medically qualified staff.

7. The medical treatment system according to one of the preceding claims, wherein the request code and/or the enabling code is/are exchanged by means of a wireless or wired communication network.

8. The medical treatment system according to one of the preceding claims, wherein furthermore, a requested treatment can be locally enabled on the medical treatment device.

9. The medical treatment system according to one of the preceding claims, wherein the enabling code generating device decides, as a function of a received request code and stored data, whether a requested treatment is plausible for the patient and an enabling code will only then be generated when the requested treatment is plausible for the patient.

10. The medical treatment system according to claim 9, wherein the decision as to whether a requested treatment is plausible includes:
- comparing the requested treatment with previous treatments and/or parameters thereof, wherein, if the treatments or parameters thereof differ substantially from each other, the requested treatment is not plausible, and/or
- checking the medical plausibility by means of an expert system wherein, when the treatment contravenes medical guidelines, the requested treatment is not plausible.

11. The medical treatment system according to one of the preceding claims, wherein the medical treatment device is a device selected from the group comprising a dialysis device, a plasmapheresis device, an apheresis device, haemodiafiltration, haemoultrafiltration, haemoperfusion, peritoneal dialysis, albumin dialysis.

12. A method for a medical treatment system (1) comprising a medical treatment device (10) and a therapy-enabling device (20), comprising the following steps:
- selecting a treatment (100) on the treatment device (10),
- generating a patient- and treatment-related request code (110), whereby the request code comprises patient identity data, allowing for a unique identification of the patient, and presenting the request code (120) via the treatment device (10) on a display (13),
- reading in the request code from the display respectively inputting the request code by means of a smartphone by a user and sending the request code read from the display or inputted via the smartphone to the therapy-enabling device (20),
- receiving the request code (130) on the therapy-enabling device (20),
- deciding (140), via the therapy-enabling device (20) as a function of a received request code and stored data, whether an enabling code will be generated, and
- sending the generated enabling code (150) via the therapy-enabling device (20),
- receiving the generated enabling code (160) on the medical treatment device (10) via means for inputting an enabling code (11, 14),
- deciding (170) by the medical treatment device (10) depending at least on the enabling code and the previously generated request code, whether and if necessary how the requested treatment will be enabled, whereby after enabling the medical treatment device (10) is unlocked, for that then the selected treatment may be executed.

13. A method for a medical treatment device (10) in a medical treatment system (1) comprising the medical treatment device (10) and a therapy-enabling device (20), comprising the following steps:
- receiving a selection (100) of treatment on the medical treatment device (10),
- generating a patient- and treatment-related request code (110), whereby the request code comprises patient identity data, allowing for a unique identification of the patient, and presenting the request code (120) via the medical treatment device (10) on the display (13),
- receiving (160) an enabling code generated by the therapy-enabling device (20) on the medical treatment device (10) via means for inputting an enabling code (11, 14),
- deciding (170) by the medical treatment device (10) depending on the enabling code and the previously generated request code, whether the requested treatment will be enabled, whereby after enabling the medical treatment device (10) is unlocked, for that then the selected treatment may be executed.

14. A method for a therapy-enabling device (20) in a medical treatment system (1) comprising a medical treatment device (10) having a display (13) and the therapy-enabling device (20), comprising the steps:
- receiving (130) a request code at the therapy-enabling device (20), whereby the request code comprises patient identity data, allowing for a unique identification of the patient, whereby the request code is read or inputted beforehand by a user via a smartphone from the display (13) and the read from the display (13) or inputted request code is send to the therapy-enabling device (20) via the smartphone,
- deciding (140) depending on a received request code and stored data, whether an enabling code will be generated, and
- sending (150) the generated enabling code via the therapy-enabling device (20).

15. A computer program product for programming devices in a medical treatment system in order to carry out steps of a method as claimed in claim 13.

16. A computer program product for programming devices in a medical treatment system in order to carry out steps of a method as claimed in claim 14.

## Revendications

1. Système de traitement médical (1) présentant
• un appareil de traitement médical (10), dans lequel l'appareil de traitement médical présente des moyens d'entrée (11) permettant de sélectionner un traitement, dans lequel l'appareil de traitement médical présente un appareil (12) pour générer un code de demande relatif au patient et au traitement, dans lequel le code de demande comprend des données d'identité du patient qui permettent une identification unique du patient, dans lequel la demande de code est fournie sur un écran (13) sur l'appareil de traitement médical (10),
• un appareil d'administration de thérapie (20), dans lequel l'appareil d'administration de thérapie (20) présente un dispositif de génération de code d'administration (22), dans lequel le dispositif de génération de code d'administration (22) est un ordinateur ou un microprocesseur, un microcontrôleur, un ASIC (Circuit intégré spécifique à l'application) configuré en conséquence ou un FPGA (Réseau de portes programmable in situ), dans lequel l'appareil d'administration de thérapie (20) présente un dispositif de réception (21) des données du code de demande, dans lequel le code de demande est lu ou scanné à l'écran par un utilisateur au moyen d'un smartphone et peut être envoyé au moyen du smartphone à l'appareil d'administration de thérapie (20), dans lequel en outre le dispositif de génération de code d'administration (22) de l'appareil d'administration de thérapie (20) décide, en fonction du code de demande reçu et des données mémorisées (DB), si un code d'administration est généré et envoie le code d'administration généré (23),
• dans lequel l'appareil de traitement médical (10) comprend en outre des moyens de lecture d'un code d'administration (11, 14),
• dans lequel l'appareil de traitement médical (10) décide au moins en fonction du code d'administration si et, le cas échéant, comment le traitement est administré, et
• dans lequel l'appareil de traitement médical (10) est conçu de telle sorte qu'après administration l'appareil de traitement médical (10) est déverrouillé afin que le traitement sélectionné puisse ensuite être effectué.

2. Système de traitement médical selon la revendication 1, dans lequel le code de demande comprend des données sélectionnées dans un groupe présentant : des données relatives à l'état du patient, des paramètres de traitement, des paramètres d'appareil, des paramètres de consommables.

3. Système de traitement médical selon une des revendications précédentes, dans lequel le code de requête est sélectionné dans un groupe présentant un code QR, un code-barres, un code Flickr.

4. Système de traitement médical selon une des revendications précédentes, dans lequel le code de demande présente un cryptage.

5. Système de traitement médical selon une des revendications précédentes, dans lequel le moyen d'entrée est sélectionné dans un groupe présentant un lecteur de cartes, une interface graphique, un lecteur de communication en champ proche.

6. Système de traitement médical selon une des revendications précédentes, dans lequel dans le cas où un traitement demandé est refusé, le code d'administration comprend une demande d'émission d'un code de demande qualifié par du personnel médicalement formé.

7. Système de traitement médical selon une des revendications précédentes, dans lequel le code de demande et/ou le code d'administration sont échangés au moyen d'un réseau de communication sans fil ou filaire.

8. Système de traitement médical selon une des revendications précédentes, dans lequel un traitement demandé peut être administré localement sur l'appareil de traitement médical.

9. Système de traitement médical selon une des revendications précédentes, dans lequel le dispositif de génération de code d'administration décide, en fonction d'un code de demande reçu et des données mémorisées, si un traitement demandé est plausible pour le patient et un code d'administration est uniquement généré si le traitement demandé est plausible pour le patient.

10. Système de traitement médical selon la revendication 9, dans lequel la décision de savoir si un traitement demandé est plausible comprend
• la comparaison du traitement demandé avec les traitements antérieurs et/ou leurs paramètres, dans lequel si les traitements ou leurs paramètres diffèrent fortement les uns des autres, le traitement demandé n'est pas plausible et/ou
• l'examen de la plausibilité médicale par un système expert, dans lequel si le traitement contredit les directives médicales, le traitement demandé n'est pas plausible.

11. Système de traitement médical selon une des revendications précédentes, dans lequel l'appareil de traitement médical (10) est sélectionné dans le groupe présentant une machine de dialyse, une machine de plasmaphérèse, une machine d'aphérèse, une hémodiafiltration, une hémo ultrafiltration, une hémoperfusion, une dialyse péritonéale, une dialyse d'albumine.

12. Procédé pour un système de traitement médical (1) présentant un appareil de traitement médical (10) et un dispositif d'administration de thérapie (20), présentant les étapes consistant à :
• sélectionner un traitement (100) sur l'appareil de traitement (10),
• générer un code de demande (110) relatif au patient et au traitement, dans lequel le code de demande présente des données d'identité du patient qui permettent une identification unique du patient, et fournir le code de demande (120) par l'appareil de traitement (10) sur un écran (13),
• lire le code de demande à l'écran ou entrer le code de demande au moyen d'un smartphone par un utilisateur et envoyer le code de demande lu ou scanné à l'écran au moyen du smartphone au dispositif d'administration de thérapie (20),
• recevoir le code de demande (130) sur l'appareil d'administration de thérapie (20),
• décider (140) par l'appareil d'administration de thérapie (20) en fonction d'un code de demande reçu et des données mémorisées si un code d'administration est généré, et
• envoyer le code d'administration généré (150) par l'appareil d'administration de thérapie (20),
• recevoir le code d'administration généré (160) sur l'appareil de traitement médical (10) via des moyens de lecture d'un code d'administration (11, 14),
• décider (170) par l'appareil de traitement médical (10) en fonction d'au moins le code d'administration et du code de demande généré précédemment, si et le cas échéant comment le traitement demandé a été administré, dans lequel après administration l'appareil de traitement médical (10) est déverrouillé afin que le traitement sélectionné puisse ensuite être effectué.

13. Procédé pour un appareil de traitement médical (10) dans un système de traitement médical (1) présentant l'appareil de traitement médical (10) et un appareil d'administration de thérapie (20), présentant les étapes consistant à :
• recevoir une sélection (100) d'un traitement sur l'appareil de traitement médical (10),
• générer un code de demande relatif à un patient et un traitement (110), dans lequel le code de demande comprend des données d'identité du patient qui permettent une identification unique du patient et fournir le code de demande (120) par l'appareil de traitement médical (10) sur un écran (13),
• recevoir (160) un code d'administration généré par l'appareil d'administration de thérapie (20) sur l'appareil de traitement médical (10) via des moyens de lecture d'un code d'administration (11, 14),
• décider (170) par l'appareil de traitement médical (10) en fonction du code d'administration et du code de demande généré précédemment, si le traitement demandé est administré, dans lequel, après administration, l'appareil de traitement médical (10) est déverrouillé afin que le traitement sélectionné puisse ensuite être effectué.

14. Procédé pour un appareil d'administration de thérapie (20) dans un système de traitement médical (1) présentant un appareil de traitement médical (10) avec un écran (13) et l'appareil d'administration de thérapie (20), présentant les étapes consistant à :
• recevoir (130) un code de demande sur l'appareil d'administration de thérapie (20), dans lequel le code de demande comprend des données d'identité du patient permettant une identification unique du patient, dans lequel le code de demande a d'abord été lu ou scanné à l'écran (13) par un utilisateur au moyen d'un smartphone et le code de demande lu ou scanné à l'écran (13) a été envoyé au moyen du smartphone à l'appareil d'administration de thérapie (20),
• décider (140) en fonction d'un code de demande reçu et des données mémorisées, si un code d'administration est généré, et
• envoyer (150) le code d'administration généré par l'appareil d'administration de thérapie (20).

15. Produit de programme informatique pour la programmation de dispositifs d'un système de traitement médical pour exécuter les étapes d'un procédé selon la revendication 13.

16. Produit de programme informatique pour la programmation de dispositifs d'un système de traitement médical pour exécuter les étapes d'un procédé selon la revendication 14.
